# EUROPEAN PATENT APPLICATION

(11) **EP 3 460 463 A1**
(43) Date of publication of application: **27.03.2019**
(21) Application number: 18160783.9
(22) Date of filing: 08.03.2018
(51) Int. Cl.: G01N 27/414

(54) **MOLECULAR DETECTION APPARATUS AND METHOD OF DETECTING MOLECULES**

(30) Priority: 20.09.2017 JP 2017180388
(71) Applicant: KABUSHIKI KAISHA TOSHIBA, Minato-ku Tokyo 105-8001 (JP)
(72) Inventor: ATSUTA, Masaki, Tokyo, 105-8001 (JP); YAMADA, Ko, Tokyo, 105-8001 (JP); NAKAMURA, Hiroko, Tokyo, 105-8001 (JP); OKI, Mitsuhiro, Tokyo, 105-8001 (JP); MIYAMOTO, Hirohisa, Tokyo, 105-8001 (JP); SHINJO, Yasushi, Tokyo, 105-8001 (JP); YOSHIMURA, Reiko, 105-8001, Tokyo (JP)
(74) Representative: Moreland, David

(57) **Abstract**

A molecular detection apparatus, comprising: a chamber; a light source provided in the chamber and configured to emit light; a detector including at least one sensor and configured to generate a first detection data and a second detection data, the sensor being provided in the chamber and being configured to capture molecules of target molecules, the first detection data corresponding to the number of captured molecules per predetermined time under a first emission condition of the light, and the second detection data corresponding to the number of captured molecules per predetermined time under a second emission condition of the light; and a discriminator to discriminate the target molecules using the first and second detection data.

## Description

### FIELD

Arrangements described herein generally relate to a molecular detection apparatus and a method of detecting molecules.

### BACKGROUND

A water heater or the like for household use is provided with an apparatus that detects carbon monoxide generated when incomplete combustion occurs and notifies the risk thereof at an early stage. Such a gas component considerably affects a human body. Although various methods have been known as a method of detecting a gas component having a relatively higher concentration, the detection methods have been limited for detecting the gas component having a concentration at ppb (parts per billion) to ppt (parts per trillion), which corresponds to an extremely low concentration.

At a disaster site, it has been desired to sense the risk in advance by detecting an extremely small amount of the gas component. The gas component having an extremely low concentration is often detected by use of large equipment in research facilities. In this case, a large-sized installation type apparatus, which is expensive and has large weight and volume, such as a gas chromatography or a mass spectrometer is required. Under such circumstances, it has been required to provide an apparatus that is capable of detecting the gas component having the extremely low concentration in real time, in other words, an apparatus that has a smaller weight and volume and a better portability and enables selective and higher sensitive detection of the gas component having the extremely low concentration in the order of ppt to ppb.

### RELEVANT REFERENCES

Patent Reference
Reference1: JPA 2010-019688
Reference 2: JPA 2010-139269
Reference 3: JPA 2015-515622

### BRIEF DESCRIPTION OF THE DRAWINGS

Fig. 1 is a block diagram illustrating a configuration example of a molecular detection apparatus.
Fig. 2 is a cross-sectional schematic view illustrating a structure example of a detector.
Fig. 3 is a cross-sectional schematic view illustrating a structure example of a sensor.
Fig. 4 is a top schematic view illustrating a structure example of a sensor chip.
Fig. 5 is a cross-sectional schematic view illustrating a structure example of a part of the detector.
Fig. 6 is a cross-sectional schematic view illustrating another structure example of the part of the detector.
Fig. 7 is a cross-sectional schematic view illustrating another structure example of the part of the detector.
Fig. 8 is a cross-sectional schematic view illustrating another structure example of the part of the detector.
Fig. 9 is a cross-sectional schematic view illustrating another structure example of the part of the detector.
Fig. 10 is a cross-sectional schematic view illustrating a structure example of the part of the detector.
Fig. 11 is a cross-sectional schematic view illustrating a structure example of the part of the detector.
Fig. 12 is a flowchart for explaining a molecular detection method example.
Fig. 13 is a schematic diagram illustrating examples of detected strengths of a substance X.
Fig. 14 is a schematic diagram illustrating examples of detected strengths of a substance Y.
Fig. 15 is a schematic diagram illustrating examples of detected strengths of a substance Z.
Fig. 16 is a top schematic view illustrating another structure example of the sensor chip.
Fig. 17 is a top schematic view illustrating another structure example of the sensor chip.
Fig. 18 is a block diagram illustrating another configuration example of the molecular detection apparatus.
Fig. 19 is a flowchart for explaining another example of the molecular detection method.
Fig. 20 is a flowchart for explaining another example of the molecular detection method.
Fig. 21 is a flowchart for explaining another example of the molecular detection method.

### DETAILED DESCRIPTION

A molecular detection apparatus, comprising: a chamber; a light source provided in the chamber and configured to emit light; a detector including at least one sensor and configured to generate a first detection data and a second detection data, the sensor being provided in the chamber and being configured to capture molecules of target molecules, the first detection data corresponding to the number of captured molecules per predetermined time under a first emission condition of the light, and the second detection data corresponding to the number of captured molecules per predetermined time under a second emission condition of the light; and a discriminator to discriminate the target molecules using the first and second detection data.

Hereinafter, arrangements will be explained with reference to the drawings. In the arrangements, substantially the same constituent portions are denoted by the same signs and explanation thereof will be omitted in some case. The drawings are schematic, and a relation between the thickness and the planar dimension of each part, a thickness ratio among parts, and so on may differ from actual ones.

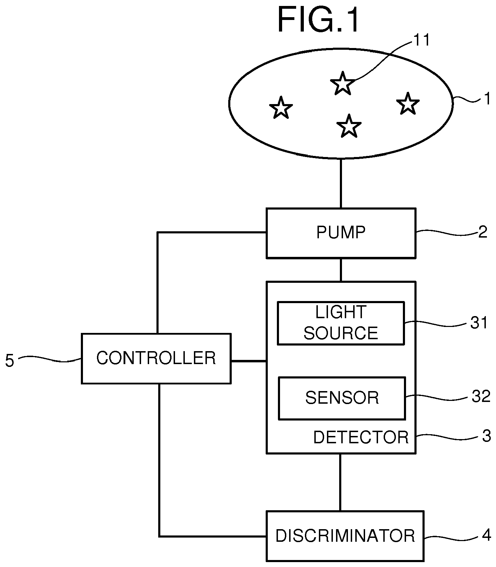

Fig. 1 is a block diagram illustrating a configuration example of a molecular detection apparatus in the arrangement. The molecular detection apparatus illustrated in Fig. 1 is, for example, an apparatus that detects molecules to be detected (substances to be detected) 11 in a detection target gas 1 generated from a gas generation source, and includes a pump 2, a detector (molecule detector) 3, a discriminator 4, and a controller 5.

The pump 2 introduces the detection target gas 1 containing the molecules to be detected 11 into the detector 3. Note that a valve may be provided in place of the pump 2 so that start and stop of the introduction of the detection target gas 1 is controlled by opening and closing the valve. Further, the detection target gas 1 may be collected by a collector or the like. The collector has a collection port for the detection target gas 1 and is connected to the pump 2 via a gas flow path. The collector may include a filter that removes impurities such as fine particles contained in the detection target gas 1. Note that the pump 2 does not always have to be provided.

The detection target gas 1 sometimes contains, as impurities, substances having a molecular weight, a molecular structure or the like similar to those of the molecules to be detected 11. Further, the molecules to be detected 11 drifting in the air often exist in a state where the molecules to be detected 11 are mixed with various foreign substances such as odor components and fine particles. From those perspectives, the detection target gas 1 may be sent to the molecular detection apparatus after being preprocessed by a filter device, a molecular distribution device, and the like beforehand.

For the filter device of the preprocessing device, a generally-used moderate-to-high performance filter or the like is used. The filter device removes particulate substances such as fine particles contained in the detection target gas 1. The detection target gas 1, from which the particulate substances have been removed in the filter device, is then sent to the molecular distribution device. An example of the molecular distribution device can be a device that ionizes the detection target gas 1 to form an ionized substance group, applies voltage to the ionized substance group to allow the ionized substance group to fly at a speed proportional to the mass thereof, and separates an ionized substance of the molecule to be detected 11 from the ionized substance group using a flight speed based on the difference in mass and a time of flight based on the flight speed. As the molecular distribution device as above, a device including an ionizer, a voltage applicator, and a time-of flight separator is used.

The detection target gas 1 containing the molecules to be detected 11 is collected by the collector directly, or after being preprocessed by the devices such as the filter device and the molecular distribution device. The molecules to be detected 11 collected by the collector are sent to the detector 3 via the gas flow path.

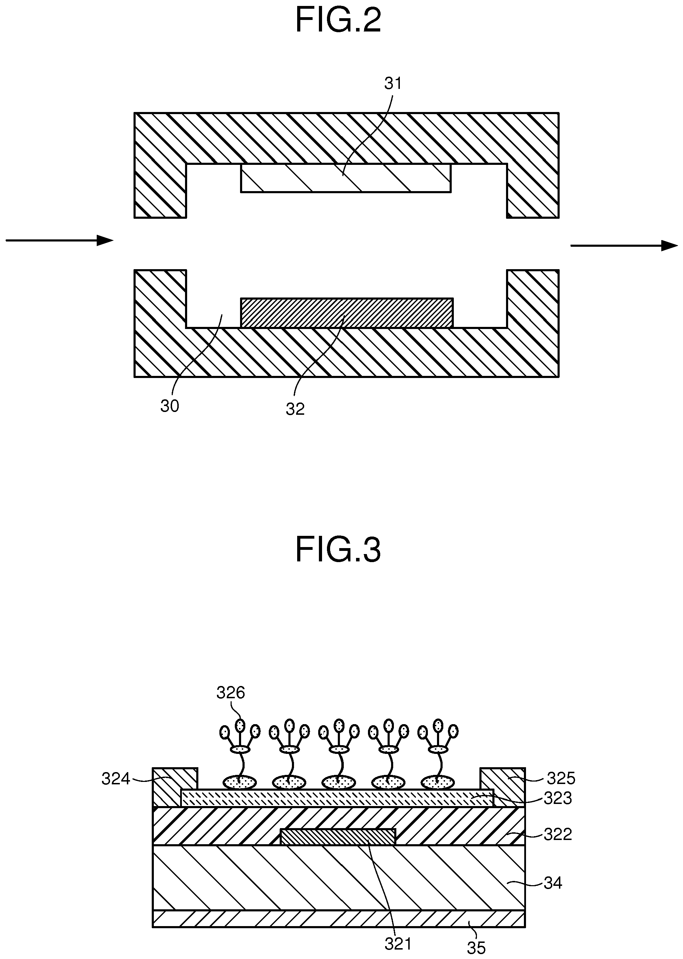

Fig. 2 is a cross-sectional schematic view illustrating a structure example of the detector 3. The detector 3 illustrated in Fig. 2 includes a measurement chamber 30, a light source 31 which is provided in the measurement chamber 30 and applies light, and a sensor 32 provided in the measurement chamber 30. The light from the light source 31 is applied to at least a part of the sensor 32.

The measurement chamber 30 is a space where the detection target gas 1 flows. Arrows illustrated in Fig. 2 indicate a flow direction of the detection target gas 1. An inner wall of the measurement chamber 30 is composed of, for example, a material that absorbs light. Note that in the case of providing the valve in place of the pump 2, the measurement chamber 30 may be reduced in pressure in advance.

The light source 31 may include, but not limited to, for example, an electric bulb, a light-emitting diode or the like. The wavelength of the light from the light source 31 can be appropriately set according to the usage. The above light may be, for example, X-ray or terahertz light and is not limited to light in a specific wavelength range. The light source 31 is provided above the sensor 32 in Fig. 2, but not limited to this.

The sensor 32 has at least one sensor. The sensor captures one or more kinds of molecules to be detected 11. The sensor 32 generates first detection data corresponding to the number of captured molecules to be detected 11 per predetermined time under a first emission condition of the light from the light source 31 and second detection data based on the number of captured molecules to be detected 11 per predetermined time under a second emission condition of the light, using the above-described sensor, and outputs them as detection signals.

The first detection data corresponds, for example, to the number of capture under non-emission or block of the light as the first emission condition, and the second detection data corresponds, for example, to the number of capture under emission of at least a part of the light as the second emission condition. Examples of the at least part of the light include all of the light, light having some wavelengths of the light, attenuating light of the light and so on. Besides, the first detection data may correspond to the number of capture under emission of a part of the light as the first emission condition, and the second detection data may correspond to the number of capture under emission of another part of the light as the second emission condition.

Fig. 3 is a cross-sectional schematic view illustrating a structure example of the sensor. The sensor includes a graphene field effect transistor (GFET) having an electrode 321, an insulating layer 322, a graphene layer 323, an electrode 324, and an electrode 325. The GFET is provided on a substrate 34. Note that the sensor may have a plurality of GFETs.

The electrode 321 overlaps with the graphene layer 323. The electrode 321 has a function as a gate electrode. The electrode 321 is formed using, for example, an indium tin oxide (ITO) or the like. The electrode 321 does not always have to be provided. Besides, the substrate 34 such as a semiconductor substrate may function as the gate electrode in place of the electrode 321.

The insulating layer 322 has a function as a gate insulating layer. Examples of the insulating layer 322 include a silicon oxide film and so on. The silicon oxide film is formed by the chemical vapor deposition (CVD) method or the like. In the case where the electrode 321 is composed of ITO, the silicon oxide film may be formed by the plasma CVD method.

The graphene layer 323 has a function as a channel formation region.

The electrode 324 and the electrode 325 are in contact with the graphene layer 323. The electrode 324 and the electrode 325 have functions as a source electrode and a drain electrode. Note that which of the electrode 324 and the electrode 325 is the source electrode or the drain electrode changes depending on the magnitude relation between potentials applied to the electrode 324 and the electrode 325.

Further, the sensor may have organic probes 326 decorated on the surface of the graphene layer 323. Note that in the case where the graphene layer 323 has a function of capturing the molecules to be detected 11, the organic probes 326 do not always have to be provided. The molecules to be detected 11 are captured by the organic probes 326, the potential distribution near the surface of the graphene layer 323 changes, and the electric field strength in the graphene layer 323 changes, thereby performing electric detection. Thus, the molecules to be detected 11 are detected.

An organic substance forming the organic probes 326 has a property of dissolving in a solvent. Thus, the organic probes 326 can be set on the graphene layer 323 by applying a solution obtained by dissolving the organic substance in a solvent to the graphene layer 323. In order to easily obtain an interaction with graphene, the organic probe 326 preferably has a portion having such a structure as a pyrene ring. A molecule having such a structure as the pyrene ring interacts with a hexagonally shaped π electron system formed by carbon of the graphene, and forms an interaction state of so-called π-π stacking. Low-concentration probe molecules are dissolved in a solvent and the resultant is applied to graphene, and thereby the π-π stacking is formed between the pyrene ring and the graphene and the probe molecules are aligned and fixed on the graphene. By using such a self-alignment action, the organic probes 326 can be set on the graphene layer 323. The organic compound forming the organic probes 326 will be described later in detail.

When the molecules to be detected 11 are captured by the organic probes 326 provided on the graphene layer 323, an output from the GFET changes. When the number of graphene layers increases to two or three, a band gap may be generated, in which case electric detection of change in electric field intensity can be performed with higher sensitivity in some cases. Therefore, the graphene layer 323 is not limited to a single layer structure of graphene but may be composed of a stack of graphene in about two layers or more and five layers or less. Besides, the graphene layer 323 may be composed using a nanoribbon of graphene to make a channel thinner so as to form a band gap. The band gap may be formed by making the graphene layer 323 in a mesh shape. Not limited to the above, the band gap may be formed in the graphene layer 323 by other methods.

The molecule to be detected 11 flying to the vicinity of the organic probe 326 is attracted to the organic probe 326 by hydrogen bonding force or the like, or comes into contact with the organic probe 326 in some cases. When the contact of the molecule to be detected 11 occurs, an interchange of electrons occurs between the molecule to be detected 11 and the organic probe 326, and the organic probe 326 transmits an electrical change to the graphene layer 323 being in contact therewith. The electrical change transmitted from the organic probe 326 to the graphene layer 323 changes the flow of electricity between the electrode 324 and the electrode 325, and thus the GFET functions as a sensor.

With the GFET using the graphene layer 323 as a channel, even an extremely slight electrical change significantly appears as an output. Accordingly, it is possible to constitute a highly sensitive sensor. In the sensor using the GFET, the electrical resistance between the electrode 324 and the electrode 325 changes due to the change in electric field intensity of the graphene layer 323 even if no potential is applied to the electrode 321. Therefore, the GFET functions as a sensor as it is. However, normally, the GFET passes electric current between the electrode 324 and the electrode 325 in a state of applying potential to the gate electrode 321, and observes an electrical change of the electrode 323 when the organic probe 326 captures the molecule to be detected 11. Note that though photocurrent flows in some cases when light is applied to the GFET, the value of the photocurrent is a negligibly small value depending on the light emission condition. Further, even with large photocurrent, discrimination of the molecule to be detected 11 using a later-described data pattern recognition method is possible by handling the photocurrent itself as a part of data.

In the detection of the molecule to be detected 11, as the change in electric field intensity of the graphene layer 323 by the molecule to be detected 11 captured by the organic probe 326 is higher, the function as the sensor is further increased. The sensor using the GFET is regarded as the most sensitive FET sensor, and can improve the sensitivity about three times as compared to a sensor using a carbon nanotube. Thus, using the sensor in which the GFET and the organic probe 326 are combined enables higher sensitive detection of the molecule to be detected 11.

At least one sensor may be provided with a plurality of organic probes 326 different in bond strength with the molecule to be detected 11. The plurality of organic probes 326 each have an interaction with the molecule to be detected 11 but are different in bond strength with the molecule to be detected 11, and thus detection data different in value is generated. In the case where a plurality of sensors are provided, the sensors may have organic probes 326 having different bond strengths respectively.

The discriminator 4 discriminates the molecule to be detected 11 using the first detection data and the second detection data. The discriminator 4 converts, for example, the first detection data and the second detection data into strength data items, and analyzes a data pattern based on a strength difference between the strength data items. The discriminator 4 stores data patterns according to substances to be detected, and compares the data patterns with the data pattern based on the first detection data and the second detection data to thereby discriminate the molecule to be detected 11 detected by the detector 3. Such signal processing is called here a pattern recognition method. The pattern recognition method enables detection and discrimination of the molecule to be detected 11 by the data pattern peculiar to the substance to be detected, for example, as in a dactyloscopy. Therefore, a gas component (the molecule to be detected 11) having an extremely low concentration on the order of ppt to ppb can be selectively and highly sensitively detected. Note that creating the data patterns stored in the discriminator 4 in consideration of the photocurrent occurring in the GFET enables cancellation of the change in current due to the photocurrent.

The controller 5 is electrically connected to the pump 2, the detector 3, and the discriminator 4, and outputs control signals to them. The controller 5 controls, by the control signals, for example, start and stop of introduction of the detection target gas 1 to the measurement chamber 30 by the pump 2, and start and stop of emission of the light by the light source 31 to at least one sensor. The controller 5 may further control, by the control signal, discrimination of the molecule to be detected by the discriminator 4.

The discriminator 4 and the controller 5 may be constituted using, for example, hardware using a processor the like. Note that each operation may be held as an operating program in a computer-readable recording medium such as a memory or the like, and each operation may be executed by reading as needed the operating program stored in the recording medium by the hardware.

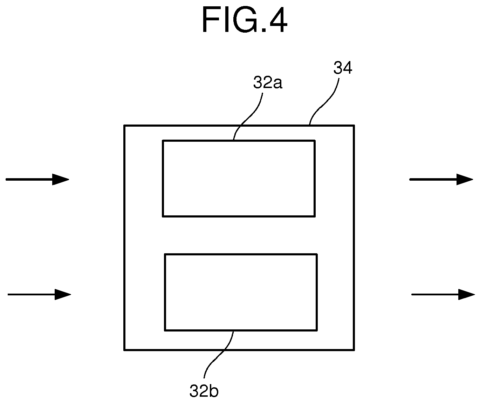

The sensor 32 may include a plurality of sensors each having a GFET. Fig. 4 is a top schematic view illustrating a configuration example of the sensor 32. The sensor 32 illustrated in Fig. 4 has a sensor 32a and a sensor 32b which are provided on the substrate 34.

The sensor 32a is provided, for example, for generating the first detection data. The sensor 32b is provided, for example, for generating the second detection data. The sensor 32a and the sensor 32b may be arranged in a grid pattern (an array pattern) or may be arranged in a linear pattern. The sensor 32a and the sensor 32b may be provided on substrates different from each other. Further, the sensor 32a and the sensor 32b may be provided in different measurement chambers from each other.

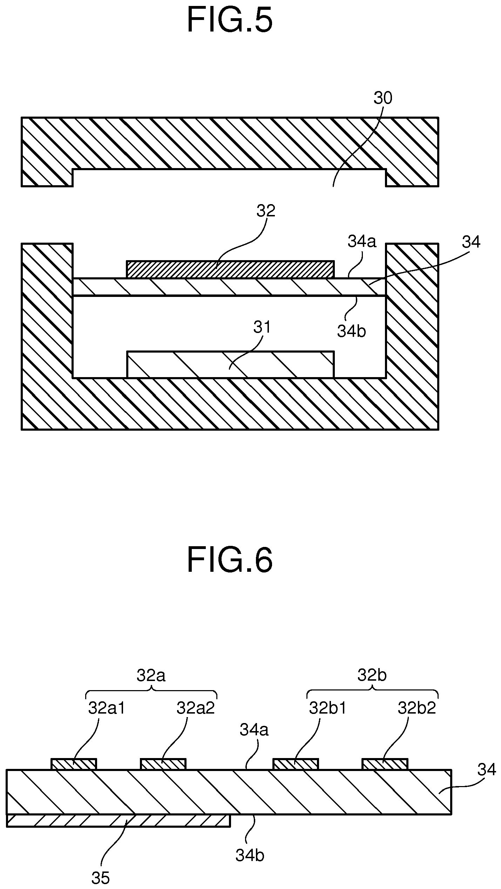

Fig. 5 is a cross-sectional schematic view illustrating a structure example of a part of the detector 3. The detector 3 illustrated in Fig. 5 includes a measurement chamber 30, a light source 31, a sensor 32, and a substrate 34. Note that for portions common to those in Fig. 2, the explanation of Fig. 2 can be cited as needed.

The substrate 34 has a surface 34a and a surface 34b on the opposite side to the surface 34a. The sensor 32 is provided on the surface 34a. The light source 31 is provided on the surface 34b side. Note that a bonding pad is provided on the sensor 32, a bonding pad for relay is provided on the substrate 34, and both of them are connected by a wire or the like, thereby making it possible to take the detection data from the sensor 32 as a signal.

The substrate 34 may be fixed to the inner wall of the measurement chamber 30. The substrate 34 can transmit the light from the light source 31. Therefore, the light can be applied from the light source 31 to the sensor 32 via the substrate 34. Examples of the substrate 34 include a quartz substrate and so on. In the case where the light transmitting property is unnecessary, a semiconductor substrate such as a silicon substrate may be used as the substrate 34.

The detector 3 may have an optical filter between the light source 31 and the sensor 32. Fig. 6 is a cross-sectional schematic view illustrating another structure example of a part of the detector 3.

The sensor 32a (sensors 32a1, 32a2) and the sensor 32b (sensors 32b1, 32b2) illustrated in Fig. 6 are provided on the surface 34a of the substrate 34, and the optical filter 35 is provided on the surface 34b of the substrate 34 and overlaps with the sensors 32a1, 32a2.

The optical filter 35 can block the light from the light source 31. This makes it possible to eliminate the emission of the light from the light source 31 to the sensors 32a1, 32a2 to thereby generate the first detection data corresponding to the number of captured molecules to be detected 11 per predetermined time under block of the light using the sensors 32a1, 32a2. As the optical filter 35 that blocks light, for example, a light blocking film such as a metal film or the like can be used.

Not limited to the above, the optical filter 35 may attenuate the light from the light source 31. Thus, the light from the light source 31 can be attenuated, namely, the emission rate of the light from the light source 31 applied to the sensors 32a1, 32a2 can be changed, so that the sensors 32a1, 32a2 can be used to generate the first detection data corresponding to the number of captured molecules to be detected 11 per predetermined time under emission of a part of the light. In this case, the optical filter 35 may be provided on the surface 34b to overlap with the sensors 32b1, 32b2. As the optical filter 35 attenuating the light, for example, an interference filter may be used which is formed, for example, by patterning a stacked film of a silicon oxide film and a silicon nitride film. Note that in the case where the optical filter 35 is provided on the surface 34b illustrated in Fig. 6, patterning or the like can be easily performed.

Not limited to the above, the optical filter 35 may absorb light having a specific wavelength of the light from the light source 31. Thus, the light having a specific wavelength of the light from the light source 31 can be absorbed and only light having other wavelengths can be applied to the sensors 32a1, 32a2, so that the sensors 32a1, 32a2 can generate the second detection data corresponding to the number of captured molecules to be detected 11 per predetermined time under emission of a part of the light. In this case, the optical filter 35 may be provided on the surface 34b to overlap with the sensors 32b1, 32b2. The optical filter 35 absorbing the light having a specific wavelength may be formed by bonding a color filter used for a liquid crystal display device or the like. Note that a color filter having a plurality kinds of transmission wavelengths may be used.

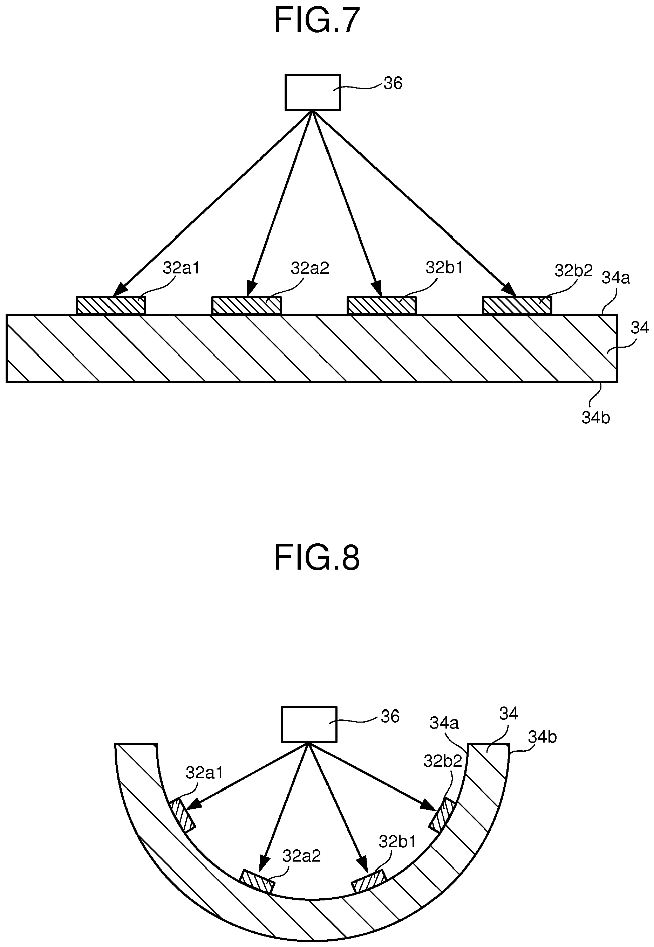

The light from the light source 31 may be dispersed and then applied to the sensor 32. Fig. 7 is a cross-sectional schematic view illustrating another structure example of the part of the detector 3. A spectroscope 36 illustrated in Fig. 7 disperses the light from the light source 31. The light source 31 may have the spectroscope 36. Rays of the dispersed light (parts of the light from the light source 31) are applied to the sensors 32a1, 32a2, 32b1, 32b2. Therefore, light rays having wavelengths different from one another are applied to the sensors 32a1, 32a2, 32b1, 32b2. Thus, the second detection data representing the spectral characteristics of the molecule to be detected 11 can be generated. As the spectroscope 36, for example, a prism, a diffraction grating or the like can be used. Note that as illustrated in Fig. 8, arrangement of the sensors 32a1, 32a2, 32b1, 32b2 on the surface 34a of the substrate 34 curved in a concave shape enables adjustment of the spaces between the sensors 32a1, 32a2, 32b1, 32b2 and the spectroscope 36 to a constant space.

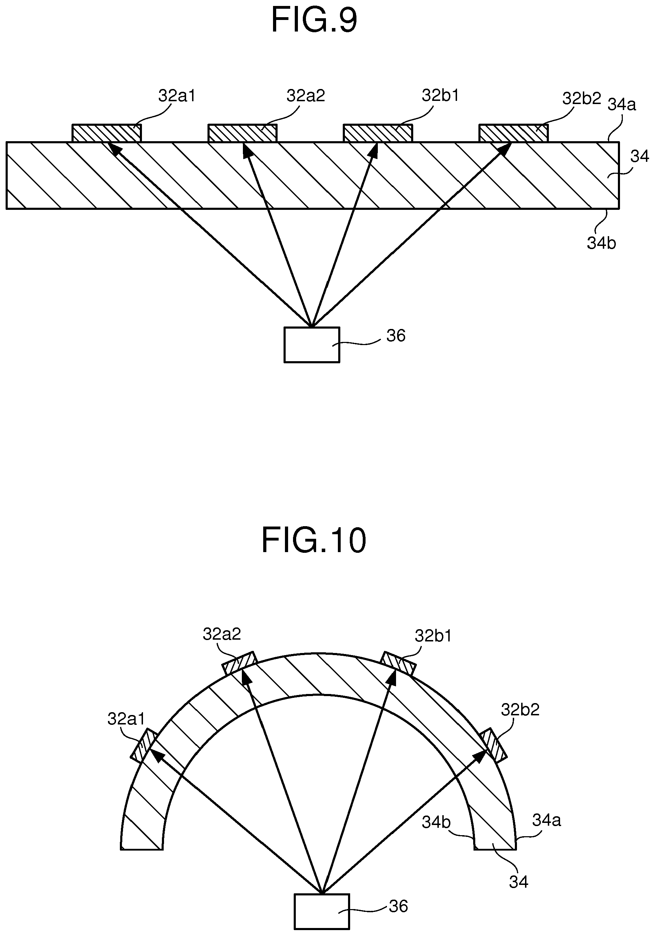

In the case of emitting the light from the light source 31 to the sensor 32 via the substrate 34, the spectroscope 36 is provided on the surface 34b side as illustrated in Fig. 9. The light source 31 may have the spectroscope 36. The rays of the dispersed light (parts of the light from the light source 31) are applied to the sensors 32a1, 32a2, 32b1, 32b2 via the substrate 34. Therefore, light rays having wavelengths different from one another are applied to the sensors 32a1, 32a2, 32b1, 32b2. Thus, the second detection data representing the spectral characteristics of the molecule to be detected 11 can be generated. As the spectroscope 36, a prism, a diffraction grating or the like can be used. Note that, as illustrated in Fig. 10, arrangement of the sensors 32a1, 32a2, 32b1, 32b2 on the surface 34a of the substrate 34 curved in a convex shape enables adjustment of the spaces between the sensors 32a1, 32a2, 32b1, 32b2 and the spectroscope 36 to a constant space.

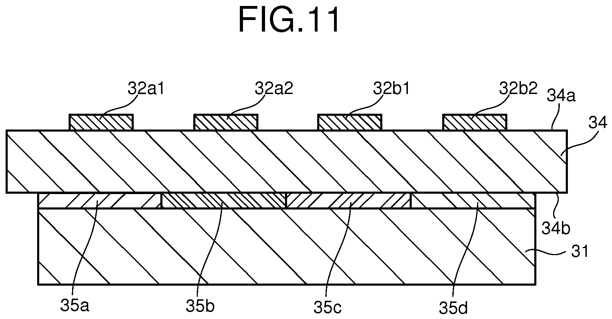

Fig. 11 is a cross-sectional schematic view illustrating another structure example of the part of the detector 3. The sensors 32a1, 32a2, 32b1, 32b2 illustrated in Fig. 11 are provided on the surface 34a, and the light source 31 is provided on the surface 34b side. The detector 3 further has an optical filter 35a to an optical filter 35d provided on the surface 34b. The light source 31 is provided on the optical filters 35a to 35d. The optical filters 35a to 35d are different in transmission wavelength from one another. Therefore, the second detection data representing the spectral characteristics of the molecule to be detected 11 can be generated by the sensors 32a1, 32a2, 32b1, 32b2.

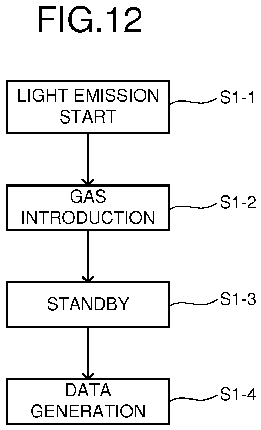

Next, as a molecular detection method example using the molecular detection apparatus in the arrangement, a molecular detection method example using the molecular detection apparatus illustrated in Fig. 1 in the case where the detector has the configuration illustrated in Fig. 5 and Fig. 6 and the optical filter 35 is the light blocking film will be explained referring to Fig. 12. Fig. 12 is a flowchart for explaining the molecular detection method example. Note that each of the sensors 32a1, 32a2, 32b1, 32b2 has the GFET and the organic probe 326 illustrated in Fig. 3, and the bond strength with the molecule to be detected 11 by the organic probe 326 is different between the sensor 32a1 and the sensor 32a2, and the bond strength with the molecule to be detected 11 by the organic probe 326 is different between the sensor 32b1 and the sensor 32b2.

The molecular detection method example illustrated in Fig. 12 includes a light emission start step (S1-1), a gas introduction step (S1-2), a standby step (S1-3), and a data generation step (S1-4). Note that the contents and order of the steps of the molecular detection method example in the arrangement are not always limited to the contents and order illustrated in Fig. 12.

In the light emission start step (S1-1), the emission of the light to the sensor 32 by the light source 31 is started on the basis of the control signal from the controller 5. The light from the light source 31 is not applied to the sensors 32a1, 32a2 which are blocked from light, but is applied to the sensors 32b1, 32b2. Accordingly, the sensors 32a1, 32a2 are placed on the emission condition under block of the light from the light source 31, whereas the sensors 32b1, 32b2 are placed on the emission condition under emission of the light from the light source 31.

In the gas introduction step (S1-2), the pump 2 introduces the detection target gas 1 into the measurement chamber 30 on the basis of the control signal from the controller 5.

In the standby step (S1-3), the molecular detection apparatus stands by in a state where the detection target gas 1 is introduced into the measurement chamber 30. A standby time is, but not particularly limited to, for example, about 3 minutes.

In the data generation step (S1-4), the sensor generates detection data. In the case of the configuration illustrated in Fig. 6, each of the sensors 32a1, 32a2 generates the first detection data under block of the light from the light source 31, and each of the sensors 32b1, 32b2 generates the second detection data under emission of the light from the light source 31.

The first detection data and the second detection data are defined by a current flowing between the source electrode and the drain electrode (also referred to as a drain current Id) when, for example, a voltage of 10 V (also referred to as a gate voltage Vg) is applied between the gate electrode and the source electrode of the GFET and a voltage of 100 mV (also referred to as a drain voltage Vd) is applied between the source electrode and the drain electrode. The gate voltage Vg may be 0 V. In this case, short-circuiting the gate electrode and the source electrode can stably set the gate voltage Vg to 0 V without providing a gate circuit. Further, setting the set value of the gate voltage Vg to near 0 V can decrease variation in characteristics due to the application of the gate voltage Vg to the GFET.

Each of the sensors 32a1, 32a2, 32b1, 32b2 captures the molecule to be detected 11. In this event, in the sensors 32b1, 32b2 under emission of light, the molecule to be detected 11 is hardly captured or the captured molecule to be detected 11 dissociates or desorbs (hereinafter, described dissociates) from the probe due to the light. Therefore, the number of captured molecules to be detected 11 is different between the sensors 32a1, 32a2 and the sensors 32b1, 32b2. In other words, the capture speed (bond and separation reaction speed) of the molecules to be detected 11 is different between the sensors 32a1, 32a2 and the sensors 32b1, 32b2. Therefore, the values of the first detection data and the second detection data are also different from each other.

The detection data may be defined by measuring a drain current Id₀ being a reference in advance and using the difference between the drain current Id₀ and the drain current Id. Further, the detection data may be defined by a value of the gate voltage Vg (referred to as a Dirac point DP here) where the drain current Id becomes smallest in an Id-Vg curve obtained by sweeping the gate voltage Vg (for example, sweeping at a step of 100 mV from -50 V to +50 V) in a state of applying the drain voltage Vd. Besides, the detection data may be defined by acquiring a Dirac point DP₀ being a reference in advance and using a difference between the Dirac point DP₀ and the Dirac point DP. Further, signal output by the detector 3 is continuously performed from before the gas introduction step (S1-2), the change of the signal is acquired as data, and the state of the change may be used as the first detection data and the second detection data for identifying the molecular structure.

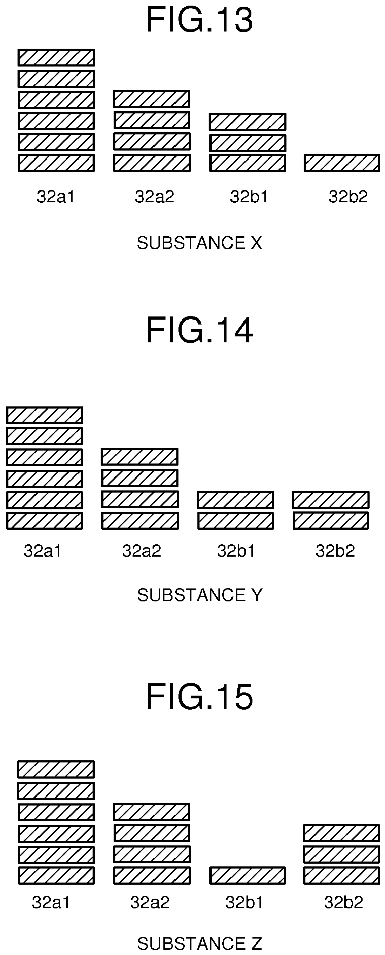

Thereafter, the discriminator 4 converts the first detection data and the second detection data into strength data items, and analyzes a data pattern based on a strength difference between the strength data items. Fig. 13 to Fig. 15 are diagrams illustrating the detected strengths of three kinds of substances X, Y, Z different from one another. As illustrated in Fig. 13 to Fig. 15, it is found that the detected strengths based on the detection data are different in the sensor 32a1, the sensor 32a2, the sensor 32b1, and the sensor 32b2, or the detected strengths are also different depending on the kinds of the substances.

The discriminator 4 discriminates the molecule to be detected 11 detected by the detector 3 by comparing the data pattern according to the substance to be detected stored in advance with the data pattern based on the first detection data and the second detection data. The above is the explanation of the molecular detection method example.

Applying the above-described pattern discrimination method makes it possible to selectively and highly sensitively detect and discriminate the molecule to be detected 11 even in a case where impurities are mixed in the detection target gas 1 introduced to the detector 3. For example, in the case where the molecule to be detected 11 is dimethyl methylphosphonate (DMMP, molecular weight: 124) which is a typical material for a toxic organophosphorus compound, there exist pesticides containing phosphoric acid such as dichlorvos having a similar chemical structure and organophosphorus pesticides, which are used often, such as malathion, chlorpyrifos, and diazinon. In order to prevent an erroneous detection of these substances, discrimination by a plurality of data patterns is effective. Specifically, since the data patterns to be detected by the sensors are different depending on the above-described substances, applying the pattern discrimination method enables selective and higher sensitive detection of the substance to be detected even if impurities having a similar molecular weight and a similar constituent element are mixed.

The molecular detection apparatus in the arrangement generates a plurality of detection data items under a plurality of environments different in emission condition of light from one another and thereby can increase the kinds of detection data. The molecular detection apparatus discriminates the molecule to be detected using many kinds of detection data and thereby can selectively and highly sensitively detect the molecule to be detected.

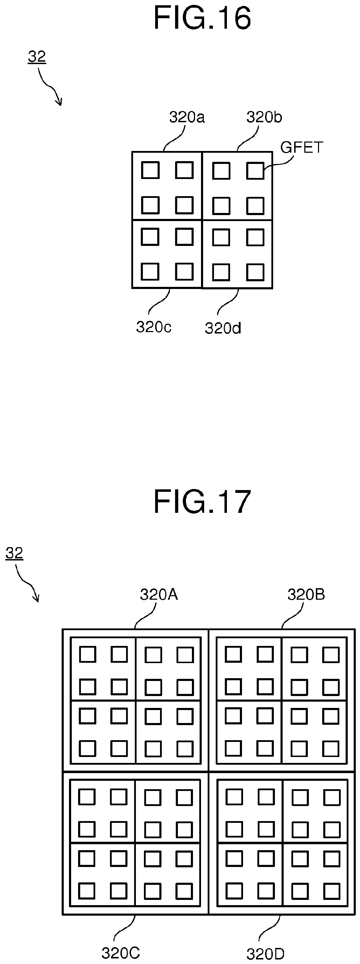

The sensor 32 may have a plurality of sensor regions different in optical characteristics. Fig. 16 is a schematic view illustrating another structure example of the sensor 32. The sensor 32 illustrated in Fig. 16 has a sensor region 320a, a sensor region 320b, a sensor region 320c, and a sensor region 320d. Each of the sensor region 320a to the sensor region 320d has at least one a sensor and may have a plurality of GFETs as illustrated in Fig. 16.

The sensors provided in the sensor region 320a to the sensor region 320d respectively may be different in at least one of transmittance and transmission wavelength of the light from the light source 31 from one another. For example, the transmittance of the light to the sensor in the sensor region 320a may be set to 0%, the transmittance of the light to the sensor in the sensor region 320b may be set to 100%, the transmittance of the light to the sensor in the sensor region 320c may be set to 1%, and the transmittance of the light to the sensor in the sensor region 320d may be set to 10%. Besides, the transmittance of the light to the sensor in the sensor region 320a may be set to 0%, the transmittance of the light to the sensor in the sensor region 320b may be set to 100%, the transmission wavelength of the light to the sensor in the sensor region 320c may be set to 300 nm, and the transmission wavelength of the light to the sensor in the sensor region 320d may be set to 400 nm. The transmittance and the transmission wavelength can be adjusted, for example, by using the above-described optical filter 35.

Fig. 17 is a schematic view illustrating another structure example of the sensor 32. The sensor 32 illustrated in Fig. 17 has a sensor region 320A, a sensor region 320B, a sensor region 320C, and a sensor region 320D, and each of the sensor region 320A to the sensor region 320D has the sensor region 320a to the sensor region 320d illustrated in Fig. 16.

At least one sensor in the sensor region 320A to the sensor region 320D has the organic probe 326 illustrated in Fig. 3 and another one sensor does not need to have the organic probe 326. For example, the sensor in the sensor region 320A may have the organic probe 326, the sensor in the sensor region 320B does not need to have the organic probe 326, the sensor in the sensor region 320C may have the organic probe 326, and the sensor in the sensor region 320D may have the organic probe 326. Further, the organic probes 326 in the sensor regions 320A, 320B, 320C, 320D may have functions of capturing molecules different from one another.

As described above, the molecular detection apparatus in the arrangement has a plurality of sensor regions having optical characteristics or capture characteristics different from one another and thereby can further increase the kinds of the detection data. Therefore, the molecular detection apparatus can selectively and highly sensitively detect the molecule to be detected using many kinds of detection data.

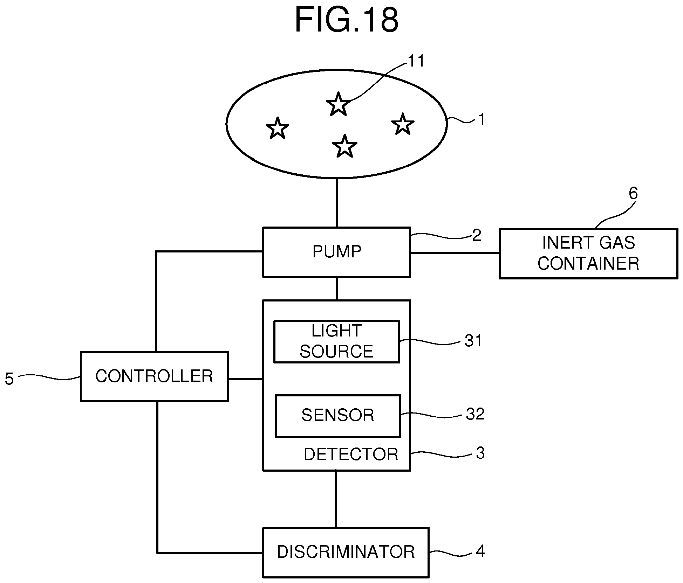

The configuration of the molecular detection apparatus in the arrangement is not limited to the configuration illustrated in Fig. 1. Fig. 18 is a schematic view illustrating another configuration example of the molecular detection apparatus.

The molecular detection apparatus illustrated in Fig. 18 includes a pump 2, a detector 3, a discriminator 4, a controller 5, and an inert gas container 6. For explanations of the detector 3, the discriminator 4, and the controller 5, the explanations of Fig. 1 to Fig. 17 can be cited as needed.

The inert gas container 6 contains an inert gas. The inert gas is preferably a gas capable of removing the molecule to be detected captured by the sensor, and its examples include nitrogen, argon and so on. The inert gas may contain hydrogen. If the detection target gas contains oxygen, hydrogen can restore the change in electric property of the GFET due to oxygen. Further, the inert gas may contain, as needed, various kinds of gas other than hydrogen. The pump 2 can switch between introduction of the detection target gas 1 to the measurement chamber 30 and introduction of the inert gas to the measurement chamber 30 from the inert gas container 6 on the basis of the control signal from the controller 5.

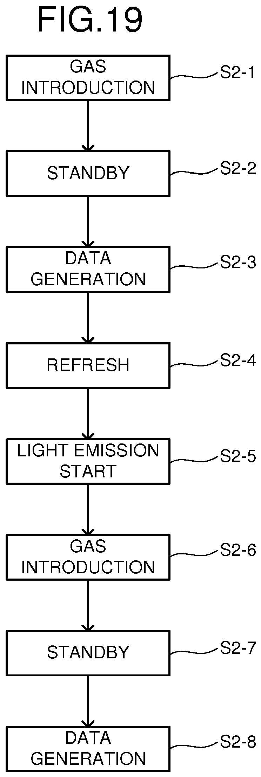

Next, as a molecular detection method example using the molecular detection apparatus illustrated in Fig. 18, a molecular detection method example in the case where the detector has the configuration illustrated in Fig. 2 and the sensor 32 has at least one sensor will be explained referring to Fig. 19. Fig. 19 is a flowchart for explaining another example of the molecular detection method. Note that the sensor has the GFET and the organic probe 326 illustrated in Fig. 3.

The molecular detection method example illustrated in Fig. 19 includes a gas introduction step (S2-1), a standby step (S2-2), a data generation step (S2-3), a refresh step (S2-4), a light emission start step (S2-5), a gas introduction step (S2-6), a standby step (S2-7), and a data generation step (S2-8). Note that the contents and order of the steps of the molecular detection method example in the arrangement are not always limited to the contents and order illustrated in Fig. 19. For portions common to those in other molecular detection method examples, the explanations of the other molecular detection method examples can be cited as needed.

In the gas introduction step (S2-1), the pump 2 introduces the detection target gas 1 into the measurement chamber 30 on the basis of the control signal from the controller 5.

In the standby step (S2-2), the molecular detection apparatus stands by in a state where the detection target gas 1 is introduced into the measurement chamber 30. A standby time is, but not particularly limited to, for example, about 3 minutes.

In the data generation step (S2-3), the sensor generates the first detection data under non-emission of the light from the light source 31.

In the refresh step (S2-4), while the pump 2 is exhausting the detection target gas 1 in the measurement chamber 30 on the basis of the control signal from the controller 5, the pump 2 introduces the inert gas into the measurement chamber 30 on the basis of the control signal from the controller 5. The introduction of the inert gas enables dissociation of at least one of the molecules to be detected 11 captured by the sensor. Note that the exhaust of the detection target gas 1 and the introduction of the inert gas may be performed alternately a plurality of times.

In the light emission start step (S2-5), the emission of the light to the sensor 32 by the light source 31 is started on the basis of the control signal from the controller 5.

In the gas introduction step (S2-6), the pump 2 introduces the detection target gas 1 into the measurement chamber 30 on the basis of the control signal from the controller 5.

In the standby step (S2-7), the molecular detection apparatus stands by in a state where the detection target gas 1 is introduced into the measurement chamber 30. A standby time is, but not particularly limited to, for example, about 3 minutes.

In the data generation step (S2-8), the sensor generates the second detection data under emission of the light from the light source 31.

Thereafter, the discriminator 4 converts the first detection data and the second detection data into strength data items, and analyzes a data pattern based on a strength difference between the strength data items. The discriminator 4 discriminates the molecule to be detected 11 detected by the detector 3 by comparing the data pattern according to the substance to be detected stored in advance with the data pattern based on the first detection data and the second detection data. The above is the explanation of the molecular detection method example.

Signal output by the detector 3 is continuously performed from before the gas introduction step (S2-1), the state of signal change is acquired as data, and the state of the change may be used as the first detection data and the second detection data for identifying the molecular structure.

The molecular detection method illustrated in Fig. 19 can generate both the first detection data and the second detection data by one sensor. Therefore, the number of sensors can be reduced. The molecular detection method illustrated in Fig. 19 can further eliminate the influence of the emission of light on the first detection data, by generating the first detection data prior to the second detection data. Further, the molecular detection method illustrated in Fig. 19 can more accurately generate the detection data corresponding to the number of captured molecules to be detected by the sensor, by introducing the refresh step. This enables improvement in accuracy of subsequent discrimination of the molecule to be detected by the discriminator 4.

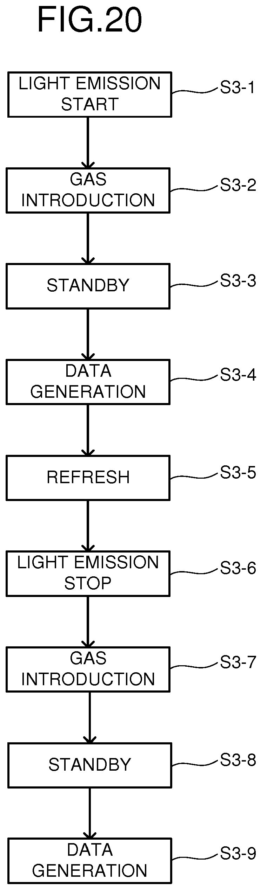

The molecular detection method using the molecular detection apparatus illustrated in Fig. 18 is not limited to the method illustrated in Fig. 19. Fig. 20 is a flowchart for explaining another example of the molecular detection method example in the case where the detector has the configuration illustrated in Fig. 2 and the sensor 32 has at least one sensor. Note that the sensor has the GFET and the organic probe 326 illustrated in Fig. 3.

The molecular detection method example illustrated in Fig. 20 includes a light emission start step (S3-1), a gas introduction step (S3-2), a standby step (S3-3), a data generation step (S3-4), a refresh step (S3-5), a light emission stop step (S3-6), a gas introduction step (S3-7), a standby step (S3-8), and a data generation step (S3-9). Note that the contents and order of the steps of the molecular detection method example in the arrangement are not always limited to the contents and order illustrated in Fig. 20. For portions common to those in other molecular detection method examples, the explanations of the other molecular detection method examples can be cited as needed.

In the light emission start step (S3-1), the emission of the light to the sensor 32 by the light source 31 is started on the basis of the control signal from the controller 5.

In the gas introduction step (S3-2), the pump 2 introduces the detection target gas 1 into the measurement chamber 30 on the basis of the control signal from the controller 5.

In the standby step (S3-3), the molecular detection apparatus stands by in a state where the detection target gas 1 is introduced into the measurement chamber 30. A standby time is, but not particularly limited to, for example, about 3 minutes.

In the data generation step (S3-4), the sensor generates the second detection data under emission of the light from the light source 31.

In the refresh step (S3-4), while exhausting the detection target gas 1 in the measurement chamber 30, the pump 2 introduces the inert gas into the measurement chamber 30 on the basis of the control signal from the controller 5. The introduction of the inert gas enables dissociation of at least one of the molecules to be detected 11 captured by the sensor. Note that the exhaust of the detection target gas and the introduction of the inert gas may be performed alternately a plurality of times.

In the light emission stop step (S3-6), the emission of the light to the sensor 32 by the light source 31 is stopped on the basis of the control signal from the controller 5.

In the gas introduction step (S3-7), the pump 2 introduces the detection target gas 1 into the measurement chamber 30 on the basis of the control signal from the controller 5.

In the standby step (S3-8), the molecular detection apparatus stands by in a state where the detection target gas 1 is introduced into the measurement chamber 30. A standby time is, but not particularly limited to, for example, about 3 minutes.

In the data generation step (S3-9), the sensor generates the first detection data under non-emission of the light from the light source 31.

Thereafter, the discriminator 4 converts the first detection data and the second detection data into strength data items, and analyzes a data pattern based on a strength difference between the strength data items. The discriminator 4 discriminates the molecule to be detected 11 detected by the detector 3 by comparing the data pattern according to the substance to be detected stored in advance with the data pattern based on the first detection data and the second detection data. The above is the explanation of the molecular detection method example.

Signal output by the detector 3 is continuously performed from before the light emission start step (S3-1), the state of signal change is acquired as data, and the state of the change may be used as the first detection data and the second detection data for identifying the molecular structure.

The molecular detection method illustrated in Fig. 20 can generate both the first detection data and the second detection data by one sensor. Therefore, the number of sensors can be reduced. Further, the molecular detection method illustrated in Fig. 20 can more accurately generate the detection data corresponding to the number of captured molecules to be detected by the sensor, by introducing the refresh step. This enables improvement in accuracy of subsequent discrimination of the molecule to be detected by the discriminator 4.

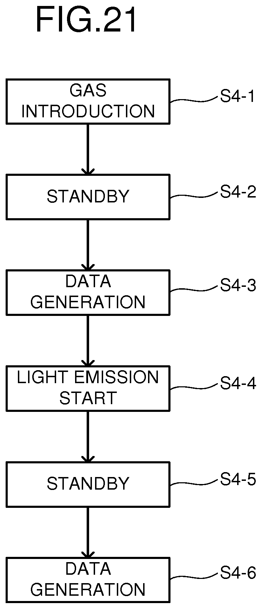

Fig. 21 is a flowchart for explaining another example of the molecular detection method. The molecular detection method example illustrated in Fig. 21 includes a gas introduction step (S4-1), a standby step (S4-2), a data generation step (S4-3), a light emission start step (S4-4), a standby step (S4-5), and a data generation step (S4-6). Note that the contents and order of the steps of the molecular detection method example in the arrangement are not always limited to the contents and order illustrated in Fig. 21. For portions common to those in other molecular detection method examples, the explanations of the other molecular detection method examples can be cited as needed.

In the gas introduction step (S4-1), the pump 2 introduces the detection target gas 1 into the measurement chamber 30 on the basis of the control signal from the controller 5.

In the standby step (S4-2), the molecular detection apparatus stands by in a state where the detection target gas 1 is introduced into the measurement chamber 30. A standby time is, but not particularly limited to, for example, about 3 minutes.

In the data generation step (S4-3), the sensor generates the first detection data under non-emission of the light.

In the light emission start step (S4-4), the emission of the light to the sensor 32 by the light source 31 is started on the basis of the control signal from the controller 5.

In the standby step (S4-5), the molecular detection apparatus stands by in a state where the detection target gas 1 is introduced into the measurement chamber 30. A standby time is, but not particularly limited to, for example, about 3 minutes.

In the data generation step (S4-6), the sensor generates the second detection data.

Thereafter, the discriminator 4 converts the first detection data and the second detection data into strength data items, and analyzes a data pattern based on a strength difference between the strength data items. The discriminator 4 discriminates the molecule to be detected 11 detected by the detector 3 by comparing the data pattern according to the substance to be detected stored in advance with the data pattern based on the first detection data and the second detection data. The above is the explanation of the molecular detection method example.

Signal output by the detector 3 is continuously performed from before the gas introduction step (S4-1), the state of signal change is acquired as data, and the state of the change may be used as the first detection data and the second detection data for identifying the molecular structure.

The molecular detection method illustrated in Fig. 21 can generate both the first detection data and the second detection data by one sensor. Therefore, the number of sensors can be reduced. Further, the molecular detection method illustrated in Fig. 21 does not perform the refresh step and therefore can generate the first detection data and the second detection data in a shorter time.

The molecular detection apparatus in the arrangement generates a plurality of detection data items under a plurality of environments different in application condition of light from one another and thereby can increase the kinds of detection data. The molecular detection apparatus discriminates the molecule to be detected using many kinds of detection data and thereby can selectively and highly sensitively detect the molecule to be detected.

Note that the molecular detection apparatus may include a heating mechanism that heats the sensor, a cooling mechanism that cools the sensor, and a pressure reducing mechanism that reduces the pressure in the measurement chamber 30. Performing heating and pressure reduction simultaneously with emission of light before measurement can remove the impurities adhering to the surface of the sensor. This makes it possible to improve the sensitivity of the sensor. Besides, at the time when the detection target gas 1 is introduced into the measurement chamber 30 and measurement is performed, emitting light and adjusting the sensor temperature of the sensor 32 enables control of the adsorption reaction speed of the molecule to be detected 11 to the surface of the sensor, thereby controlling the response characteristics with respect to the molecule to be detected 11 to characteristics suitable for measurement. Further, performing heating and pressure reduction simultaneously with the emission of light after the measurement enables removal of the molecule to be detected 11 adsorbing to the surface of the sensor. Accordingly, the sensitivity of the sensor in subsequent measurement can be improved.

The detection and discrimination results of the molecule to be detected 11 obtained in the molecular detection apparatus in the arrangement may be transmitted over an information network and utilized. For example, the molecular detection apparatus may have, attached thereto or therein, an information processer including a function of transmitting the detection information on the molecule to be detected 11 over the information network and a function of collating the detection information with reference information acquired from the information network. The information processer includes an information transmitter that transmits the detection information on the molecule to be detected 11, an information receiver that receives the reference information, and an information collator that collates the detection information with the reference information. The information processer may have only one of an information transmitting function and an information collating function including an information receiving function.

The detection information on the molecule to be detected 11 is transmitted from the information transmitter to an information user over a network. To collate the detection information on the molecule to be detected 11 with the existing reference information, the reference information is acquired by the information receiver over the network. The acquired reference information is collated with the detection information by the information collator. By acquiring information from an external network and referring to it, a function of carrying a lot of information and analyzing the information can be replaced with an external one, thereby further downsizing the molecular detection apparatus to increase the portability thereof. Further, use of a network communications tool also enables quick acquisition of new data patterns in the pattern recognition method. On the information receiving side, a next action can be taken based on this information. It is possible to dispose portable molecular detection apparatuses at places so as to collect data to be obtained from the places and analyze the data, for use in evacuation guidance under abnormal circumstances or the like. Combination of the network and the molecular detection apparatus creates a lot of use ways which have not been conventionally achieved, leading to improvement in industrial value.

while certain arrangements have been described, these arrangements have been presented by way of example only, and are not intended to limit the scope of the claims. Indeed, the magnet described herein may be embodied in a variety of other forms; furthermore, various omissions, substitutions and changes in the form of the magnet described herein may be made.

### (Numbered Clauses relating to the arrangements)

1. A molecular detection apparatus, comprising:
   a chamber;
   a light source provided in the chamber and configured to emit light;
   a detector including at least one sensor and configured to generate a first detection data and a second detection data, the sensor being provided in the chamber and being configured to capture molecules of target molecules, the first detection data corresponding to the number of captured molecules per predetermined time under a first emission condition of the light, and the second detection data corresponding to the number of captured molecules per predetermined time under a second emission condition of the light; and
   a discriminator to discriminate the target molecules using the first and second detection data.
2. The apparatus according to clause 1, further comprising:
   a pump to introduce a target gas containing the target molecules into the chamber; and
   a controller to control start and stop of introduction of the target gas into the chamber by the pump, and start and stop of emission of the light by the light source to the sensor.
3. The apparatus according to clause 2, further comprising:
   a container to accommodate an inert gas, wherein:
   the pump is configured to switch introduction of the target gas into the chamber and introduction of the inert gas into the chamber; and
   the controller is configured to control start and stop of introduction of the inert gas by the pump.
4. The apparatus according to any one of clauses 1 to 3, wherein
   the sensor includes a transistor having a graphene layer, a first electrode provided on the graphene layer, and a second electrode provided on the graphene layer.
5. The apparatus according to clause 4, wherein
   the sensor includes an organic probe disposed on the graphene layer.
6. The apparatus according to clause 5, wherein
   the sensor includes a plurality of the transistors and a plurality of the probes, the probes being disposed on the graphene layers and having different bond strengths with the molecules of the target molecules.
7. The apparatus according to any one of clauses 1 to 6, wherein:
   the first detection data corresponds to the number of the captured molecules under non-emission or block of the light of the first condition; and
   the second detection data corresponds to the number of the captured molecules under emission of at least a part of the light of the second condition.
8. The apparatus according to clause 7, wherein:
   the sensor includes a first sensor to generate the first detection data and a second sensor to generate the second detection data; and
   the detector further includes an optical filter provided between the first sensor and the light source and configured to block the light.
9. The apparatus according to any one of clauses 1 to 6, wherein:
   the sensor includes a first sensor to generate the first detection data and a second sensor to generate the second detection data; and
   the detector further includes an optical filter provided between the first or second sensor and the light source and configured to attenuate the light.
10. The apparatus according to any one of clauses 1 to 6, wherein:
   the sensor includes a first sensor to generate the first detection data and a second sensor to generate the second detection data; and
   the detector further includes an optical filter provided between the first or second sensor and the light source and configured to absorb light having a predetermined wavelength of the light.
11. The apparatus according to any one of clauses 8 to 10, wherein:
   the detector further includes a light transmissive substrate having a first surface and a second surface opposite to the first surface;
   the first and second sensors are provided on the first surface; and
   the optical filter is provided on the second surface.
12. The apparatus according to any one of clauses 1 to 6, wherein:
   the sensor includes a first sensor to generate the first detection data and a second sensor to generate the second detection data; and
   the detector further includes a spectroscope to disperse the light and from which rays of the dispersed light is emitted to the first and second sensors.
13. A method of detecting molecules, comprising:
   introducing a detection target gas containing target molecules into a chamber;
   generating a first detection data under non-emission of light after the introduction of the target gas, the first detection data corresponding to the number of molecules captured by at least one sensor per predetermined time, the sensor being provided in the chamber;
   starting emission of the light to the sensor;
   generating a second detection data under emission of the light, the second detection corresponding to the number of molecules captured by the sensor per predetermined time; and
   discriminating the target molecules using the first and second detection data.
14. The method according to clause 13 wherein:
   the emission of the light is started before the introduction of the target gas, and stopped after the introduction of the target gas and before the generation of the first detection data; and
   the second detection data is generated after the introduction of the target gas and before the stop of the emission of the light.
15. The method according to clause 13 or clause 14, further comprising:
   exhausting the target gas from the chamber and introducing an inert gas into the chamber after the generation of one of the first and second detection data to remove the captured molecules on the sensor; and
   introducing the target gas into the chamber after the removal of the captured molecules and before the generation of the other one of the first and second detection data.

## Claims

1. A molecular detection apparatus, comprising:
a chamber;
a light source provided in the chamber and configured to emit light;
a detector including at least one sensor and configured to generate a first detection data and a second detection data, the sensor being provided in the chamber and being configured to capture molecules of target molecules, the first detection data corresponding to the number of captured molecules per predetermined time under a first emission condition of the light, and the second detection data corresponding to the number of captured molecules per predetermined time under a second emission condition of the light; and
a discriminator to discriminate the target molecules using the first and second detection data.

2. The apparatus according to claim 1, further comprising:
a pump to introduce a target gas containing the target molecules into the chamber; and
a controller to control start and stop of introduction of the target gas into the chamber by the pump, and start and stop of emission of the light by the light source to the sensor.

3. The apparatus according to claim 2, further comprising:
a container to accommodate an inert gas, wherein:
the pump is configured to switch introduction of the target gas into the chamber and introduction of the inert gas into the chamber; and
the controller is configured to control start and stop of introduction of the inert gas by the pump.

4. The apparatus according to any one of claims 1 to 3, wherein
the sensor includes a transistor having a graphene layer, a first electrode provided on the graphene layer, and a second electrode provided on the graphene layer.

5. The apparatus according to claim 4, wherein
the sensor includes an organic probe disposed on the graphene layer.

6. The apparatus according to claim 5, wherein
the sensor includes a plurality of the transistors and a plurality of the probes, the probes being disposed on the graphene layers and having different bond strengths with the molecules of the target molecules.

7. The apparatus according to any one of claims 1 to 6, wherein:
the first detection data corresponds to the number of the captured molecules under non-emission or block of the light of the first condition; and
the second detection data corresponds to the number of the captured molecules under emission of at least a part of the light of the second condition.

8. The apparatus according to claim 7, wherein:
the sensor includes a first sensor to generate the first detection data and a second sensor to generate the second detection data; and
the detector further includes an optical filter provided between the first sensor and the light source and configured to block the light.

9. The apparatus according to any one of claims 1 to 6, wherein:
the sensor includes a first sensor to generate the first detection data and a second sensor to generate the second detection data; and
the detector further includes an optical filter provided between the first or second sensor and the light source and configured to attenuate the light.

10. The apparatus according to any one of claims 1 to 6, wherein:
the sensor includes a first sensor to generate the first detection data and a second sensor to generate the second detection data; and
the detector further includes an optical filter provided between the first or second sensor and the light source and configured to absorb light having a predetermined wavelength of the light.

11. The apparatus according to any one of claims 8 to 10, wherein:
the detector further includes a light transmissive substrate having a first surface and a second surface opposite to the first surface;
the first and second sensors are provided on the first surface; and
the optical filter is provided on the second surface.

12. The apparatus according to any one of claims 1 to 6, wherein:
the sensor includes a first sensor to generate the first detection data and a second sensor to generate the second detection data; and
the detector further includes a spectroscope to disperse the light and from which rays of the dispersed light is emitted to the first and second sensors.

13. A method of detecting molecules, comprising:
introducing a detection target gas containing target molecules into a chamber;
generating a first detection data under non-emission of light after the introduction of the target gas, the first detection data corresponding to the number of molecules captured by at least one sensor per predetermined time, the sensor being provided in the chamber;
starting emission of the light to the sensor;
generating a second detection data under emission of the light, the second detection corresponding to the number of molecules captured by the sensor per predetermined time; and
discriminating the target molecules using the first and second detection data.

14. The method according to claim 13 wherein:
the emission of the light is started before the introduction of the target gas, and stopped after the introduction of the target gas and before the generation of the first detection data; and
the second detection data is generated after the introduction of the target gas and before the stop of the emission of the light.

15. The method according to claim 13 or claim 14, further comprising:
exhausting the target gas from the chamber and introducing an inert gas into the chamber after the generation of one of the first and second detection data to remove the captured molecules on the sensor; and
introducing the target gas into the chamber after the removal of the captured molecules and before the generation of the other one of the first and second detection data.
